(19) Europäisches Patentamt
European Patent Office
Office européen des brevets

(11) **EP 2 345 714 B1**

(12) **EUROPEAN PATENT SPECIFICATION**

(45) Date of publication and mention
of the grant of the patent:
**12.09.2018 Bulletin 2018/37**

(21) Application number: **09821833.2**

(22) Date of filing: **23.10.2009**

(51) Int Cl.:
*C12N 5/071* (2010.01)       *C12N 5/07* (2010.01)
*A01N 1/02* (2006.01)        *C12M 1/32* (2006.01)
*C12M 1/04* (2006.01)        *C12M 1/00* (2006.01)
*C12M 3/00* (2006.01)

(86) International application number:
**PCT/JP2009/005618**

(87) International publication number:
**WO 2010/047133 (29.04.2010 Gazette 2010/17)**

(54) **CELL STORAGE METHOD AND USE THEREOF FOR CELL TRANSPORT**

ZELLAUFBEWAHRUNGSVERFAHREN UND DESSEN VERWENDUNG ZUM ZELLTRANSPORT

PROCÉDÉ DE STOCKAGE DE CELLULES ET SON UTILISATION POUR LE TRANSPORT DE CELLULES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **24.10.2008 JP 2008273846**

(43) Date of publication of application:
**20.07.2011 Bulletin 2011/29**

(73) Proprietors:
• **Kuraray Co., Ltd.**
**Okayama 710-0801 (JP)**
• **PUBLIC UNIVERSITY CORPORATION YOKOHAMA CITY UNIVERSITY**
**Yokohama-shi**
**Kanagawa 236-0027 (JP)**

(72) Inventors:
• **ITCHODA, Yoko**
**Tsukuba-shi**
**Ibaraki 305-0841 (JP)**
• **TAZAKI, Go**
**Tsukuba-shi**
**Ibaraki 305-0841 (JP)**
• **FUKUDA, Motohiro**
**Tsukuba-shi**
**Ibaraki 305-0841 (JP)**
• **TSURUTA, Hitoshi**
**Tsukuba-shi**
**Ibaraki 305-0841 (JP)**

• **TANIGUCHI, Hideki**
**Yokohama-shi**
**Kanagawa 236-0004 (JP)**

(74) Representative: **Vossius & Partner**
**Patentanwälte Rechtsanwälte mbB**
**Siebertstrasse 3**
**81675 München (DE)**

(56) References cited:
EP-A1- 1 840 207        WO-A1-2006/075597
WO-A1-2007/049576       WO-A1-2007/069666
WO-A1-2007/126127       WO-A1-2008/130025
WO-A1-2008/156041       JP-A- 2006 325 532
JP-A- 2007 228 818      JP-T- 2006 523 091
US-A- 5 858 770

• LEFKOVITS, I. ET AL.: 'Proteomic analysis of rare molecular species of translated polypeptides from a mouse fetal thymus cDNA library.' PROTEOMICS vol. 1, no. 4, 2001, pages 560 - 573, XP008146293
• AMEMIYA, C.T. ET AL.: 'Generation of a Zebrafish P1 Artificial Chromosome Library.' GENOMICS vol. 58, no. 2, 1999, pages 211 - 213, XP004449373
• TORISAWA, Y. ET AL.: 'A multicellular spheroid array to realize spheroid formation, culture, and viability assay on a chip' BIOMATERIALS vol. 28, no. 3, 2007, pages 559 - 566, XP005717776

## Description

### Technical Field

The present invention relates to a cell storage method to store living cells.

### Background Art

A technique of using cells isolated from a tissue in testing or examination is an essential method in the biotechnology-related fields. It is widely used in diagnosing a disease or pathological condition, searching for a new drug and evaluating the efficacy of a drug, or in animal inspection, plant inspection, testing for environmental pollutants, and so on. Thus, cells and the like used in the biotechnology field have been greatly diversified.

The isolated cells are sometimes used immediately for testing, but in many cases, the cells are cultured in a culture dish or a test tube. Various examinations are carried out using the cultured cells. Cell lines in culture for use in cell culture tests are required to show drug susceptibility and toxic reaction that are similar to those obtained in a test performed in a living body, that is, a so-called in vivo test. In short, it is necessary to be able to construct an intercellular network regularly arranged on the surface of a cell culture chamber. Further, the cell lines in culture for use in cell culture tests are extremely expensive, so an improvement in survival rate and proliferation rate of cells is desired.

The cell culture tests measure the effect of a drug or the like to be evaluated, by changing its amount, concentration, and the like under the same conditions. For this reason, it is necessary that the cell culture chambers be identical in material, shape, and the like. As the cell culture chambers, a petri dish made of plastic, a petri dish made of glass, a glass plate fixed into a chamber, a well plate, and the like are generally used. Examples of the well plate include 6-well, 12-well, 48-well, and 96-well plates or petri dishes. In general, the size of the entire plate is substantially the same, and as the number of wells increases, the size of a single well decreases. A single well corresponds to a single culture dish. With the recent trend toward miniaturization, a 384-well plate having a number of culture dishes with a small diameter has also come to be used. Bottoms of these culture dishes have a flat plate shape, and each of the bottom surfaces is used as a culture surface.

However, the use of the conventional cell culture chamber for culturing tissue cells causes the cells to be thinned into a form with no orientation. Additionally, the cells are randomly arranged on the surface of the cell culture chamber, so intercellular networks cross each other in a complicated manner. This causes a problem of being incapable of reproducing cell functions in vivo. In liver cells, for example, a mass of liver cells in spheroid that is known to have a function of maintaining the liver functions is not formed.

To overcome the above problems, there are disclosed a method for immobilizing particular polymers on a culture surface having a flat plate shape (see Patent Literature 1), a method using a particular apparatus (see Patent Literature 2), a method for culturing in a polymer gel (see Patent Literature 3), and the like.

However, in the method described in Patent Literature 1, stable production is impossible, since the immobilization method is complicated. This causes a problem of an increase in cost. Further, in the method described in Patent Literature 2, even though the culturing method is complicated, the formation efficiency of cell masses is low. This causes a problem of an increase in cost, for example. The method described in Patent Literature 3 has problems that the size of each cell mass cannot be controlled, and the operation of bases is complicated, for example. Therefore, these methods are complicated and incapable of stable production, and the costs increase. Furthermore, EP 1840207 describes a bioassay method wherein cultured cells are used and a cell culture for a therapeutic purpose in the case of determining the efficacy of a drug or examining the toxicity thereof. Further, EP 1840207 aims to provide a method of culturing cells and a cell culture plate.

### Citation List

### Patent Literature

Patent Literature 1: Japanese Patent No. 3177610
Patent Literature 2: Japanese Unexamined Patent Application Publication No.7-79772
Patent Literature 3: Japanese Unexamined Patent Application Publication No. 8-308562

## Summary of Invention

### Technical Problem

It is preferable to store living cells, which are isolated from a living body, in the same environment as in a living body. For example, in the case of storing non-frozen liver cells, the liver cells are adhered to a planar culture plate to be filled with a culture medium, and are stored and keep warm (for example, at a temperature of 25 to 37°C). However, when the cells are cultured in the flat plate, the environment is greatly different from that within a living body. This causes a problem of losing the original functions of the cells. There is another problem that the cell functions are deteriorated due to environmental factors during culture or storage, such as shaking, temperature, and $CO_2$ concentration (pH change in the culture medium).

Further, there is a concern that when the cells cultured by the method disclosed in Patent Literature 1 or 2 are stored, cell death is caused by cell detachment or breakup of a gel in case of shaking, or the like. Therefore, it has been difficult to transport the stored living cells while maintaining the three-dimensional structure of living cells and to use the living cells at distance from the place where the living cells are separated. On the other hand, there is a demand for storing and transporting the isolated cells without degrading the functions of the isolated cells. This is because the isolation of cells from a tissue and the testing and examination thereof are carried out in different institutions.

The present invention has been made to solve the above-mentioned problems, and therefore has an object to provide a cell storage method for storing living cells while maintaining cell functions.

### Solution to Problem

The present invention is directed to the following:

1. A cell storage method to store living cells by using a cell culture chamber including a plurality of micro spaces wherein the plurality of micro spaces have a bottom area of 0.01 to 0.1mm2 and a depth of 25 to 150μm, the cell storage method comprising the steps of:

> culturing the living cells by being adhered to surfaces of the plurality of micro spaces, wherein the culturing is carried out so that a cell population of a three-dimensional structure which is adhered to the surfaces and formed within each of the micro spaces has a number of cells that are separated from other cells;
> removing non-adherent cells after the culturing; and then
> pouring a culture medium into the cell culture chamber so as to cover the plurality of micro spaces; and
> sealing the cell culture chamber so as to prevent the culture medium from leaking from the cell culture chamber, and storing the living cells
> wherein the cell culture chamber is maintained at a temperature equal to or higher than 10°C and lower than 20°C to store the living cells, and
> wherein the living cells are one of liver cells, beta cells of pancreas, cardiac muscle cells, nerve cells, skin epidermal cells, cartilage cells, bone cells, tissue stem cells, ES cells, and iPS cells.

2. The cell storage method according to item 1 wherein the living cells are liver cells.

3. The cell storage method according to item 1, wherein the living cells are one of liver cells, beta cells of pancreas, cardiac muscle cells, nerve cells, skin epidermal cells, cartilage cells, and bone cells, which are differentiated from one of tissue stem cells, ES cells, and iPS cells.

4. The cell storage method according to any one of items 1 to 3, wherein the culturing of the living cells includes further culturing the living cells to allow the living cells to grow, elongate, or aggregate after the living cells are adhered and cultured.

5. The cell storage method according to any one of items 1 to 4, wherein the living cells are seeded in the plurality of micro spaces at a cell seeding density of $1 \times 10^2$ to $1 \times 10^6$ cells/cm2.

6. The cell storage method according to any one of items 1 to 5, wherein a cell mass having the living cells accumulated therein is formed in each of the plurality of micro spaces.

7. The cell storage method according to item 6, wherein the cell mass has a diameter of 30 to 200 μm.

8. The cell storage method according to any one of items 1 to 7, wherein the culture medium includes at least one of a blood serum, a growth factor, and a component of blood.

9. The cell storage method according to any one of items 1 to 8, wherein the cell culture chamber is sealed by using a membrane which allows oxygen or carbon dioxide to permeate.

10. Use of the method of item 1 for transporting living cells in vehicle, ship or aircraft.

As disclosed herein, the cell culture chamber may be maintained at a temperature equal to or higher than 4C° and lower than 37C° to store the living cells. Furthermore, it is preferred that the culturing be carried out so that a cell population of a three-dimensional structure which is adhered to the surfaces and formed
within each of the micro spaces has a number of cells that are separated from other cells. The use of the micro spaces prevents the living cells cultured in each of the micro spaces from contacting the living cells within other micro spaces.
The plurality of micro spaces have a bottom area of 0.01 to 0.1 mm$^2$ and a depth of 25 to 150 $\mu$m. The living cells are one of liver cells, beta cells of pancreas, cardiac muscle cells, nerve cells, skin epidermal cells, cartilage cells, bone cells, tissue stem cells, ES cells, and iPS cells. Alternatively, the living cells are preferably one of liver cells, beta cells of pancreas, cardiac muscle cells, nerve cells, skin epidermal cells, cartilage cells, and bone cells, which are differentiated from one of tissue stem cells, ES cells, and iPS cells.
It is preferred that after the culturing, a non-adhered cell be removed and then the culture medium be poured into the cell culture chamber. It is also preferred that the culturing of the living cells include further culturing the living cells to allow the living cells to grow, elongate, or aggregate after the living cells are adhered and cultured.
It is preferred that the living cells be seeded in the plurality of micro spaces at a cell seeding density of $1\times10^2$ to $1\times10^6$ cells/cm$^2$, more preferably at a cell seeding density of $1\times10^4$ to $1\times10^6$ cells/cm$^2$. It is preferred that a cell mass having the living cells accumulated therein be formed in each of the plurality of micro spaces. It is preferred that the cell mass has a diameter of 30 to 200 $\mu$m.
It is more preferred to satisfy at least one of the following conditions: the culture medium includes at least one of blood serum; a growth factor, and a component of a blood and the cell culture chamber is sealed with a membrane which allows oxygen or carbon dioxide to permeate.

**Advantageous Effects of Invention**

According to the present invention, it is possible to provide a cell storage method for storing living cells while maintaining cell functions as defined in the claims.

**Brief Description of the Drawings**

Fig. 1 is a plane view showing a structure of a cell culture chamber according to an embodiment;
Fig. 2 is a cross-sectional view along the line II-II showing the structure of the cell culture chamber according to an embodiment;
Fig. 3 is a plane view showing another structure of a cell culture chamber according to an embodiment;
Fig. 4 is a cross-sectional view along the line IV-IV showing another structure of the cell culture chamber according to an embodiment;
Fig. 5 is a plane view showing still another structure of a cell culture chamber according to an embodiment;
Fig. 6 is a cross-sectional view along the line VI-VI showing still another structure of the cell culture chamber according to an embodiment;
Fig. 7 is a view showing an exemplary state in which cells are cultured and sealed using the cell culture chamber shown in Figs. 5 and 6;
Fig. 8A is a photograph showing a result of Example 06; and
Fig. 8B is a photograph showing a result of Example 07.

**Description of Embodiments**

A cell storage method according to the present invention is a method for culturing living cells by being adhered to microchambers using a cell culture chamber which is suitable for culturing the living cells, forming three-dimensional structures in the microchambers, filling the cell culture chamber with a culture medium after the culturing, and storing it as defined in the claims. The microchambers allow the living cells to form the three-dimensional structures and maintain the structures. Further, the microchambers allow the three-dimensional structures of the living cells formed in the microchambers to be separated from other three-dimensional structures of the living cells. Therefore, there is a need to use suitable microchambers to culture the living cells. A chamber as described below is used as the cell culture chamber for the storage, for example.

A cell culture chamber has a concave-convex pattern, i.e., a plurality of microchambers formed therein. This permits cells to grow in three dimensions, like in a living body, and also permits cells to be cultured in aggregated form with no variation in each microchamber. The height of side walls (convex portions) for partitioning the microchambers is optimized, thereby making it possible to culture aggregated living cells (for example, a mass of liver cells) exclusively within the microchambers. Note that the term "micro space" refers to a space formed by a microchamber, more specifically to a space formed by a concave-convex pattern formed on a plane surface. Hereinafter, the microchamber and the micro space are not particularly distinguished from each other.

The dimensions of the microchambers each surrounded by the side walls have to be set within the optimum range for culturing cells. If the bottom area of each microchamber is too large, cells are thinly spread and fail to form a three-dimensional structure, as in the culture on a flat plate. If, on the other hand, the bottom area of each microchamber is too small, it cannot accommodate cells. Accordingly, the dimensions of the space structure are preferably in a range capable of containing one or a plurality of cells according to cell species to be cultured. In the case of forming the mass of liver cells in which a plurality of cells is accumulated, the dimensions are preferably in a range capable of containing the mass of liver cells.

The height of each side wall has to be set within the optimum range for preventing the cells cultured in the microchambers from moving to the adjacent microchambers. If the height of each side wall is too low, the cells run on the side wall, and thus such side wall is unsuitable for culture. If the height of each side wall is too high, the production thereof is difficult and material diffusion becomes difficult, leading to a deterioration of the culture environment. Therefore, the height of each side wall is preferably in the range capable of continuously and stably culturing cells, which are arranged in the microchambers according to cell species, within the microchambers.

In addition, openings are formed in the side walls to obtain a structure in which the plurality of microchambers communicates with each other, thereby making it possible to supply oxygen and nutrients to cells and remove waste products from the cells effectively. Note that the height of the side walls, the dimensions of the microchambers, and the width of the openings are appropriately set according to cell species to be cultured, thereby enabling application to various culture systems.

In this specification, the term "living cells" refers to cells (primary cultured cells) which are isolated from a living body tissue and which are not passaged. The living cells include two kinds of cells, frozen cells and fresh cells. The living cells also include cell lines, other ES cells (Embryonic Stem cells), and so on. The term "fresh cells" refers to primary cultured cells which are not frozen.

Embodiment

Hereinafter, as an example, an embodiment of the present invention is described.

Further, to clarify the explanation, the following description and the drawings are appropriately simplified.

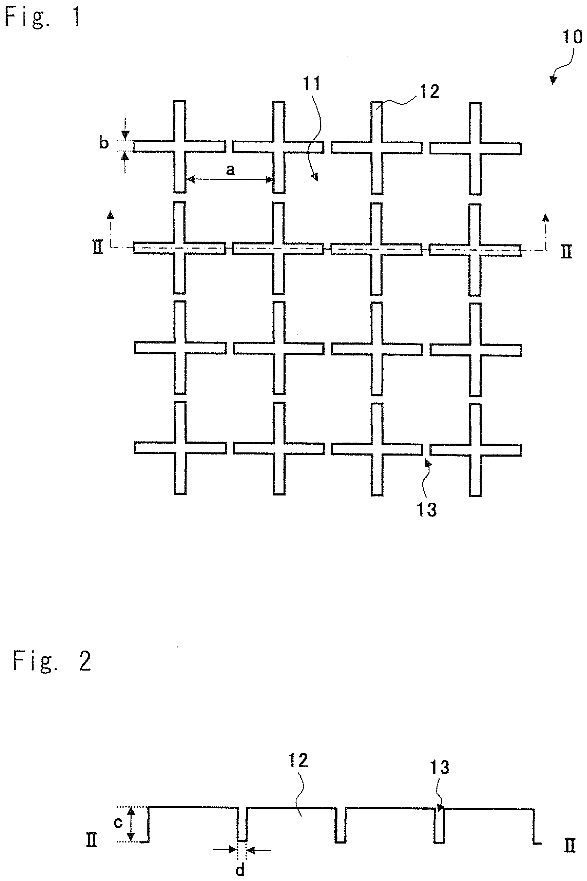

First, a cell culture chamber for use in a cell storage method according to an embodiment will be described, and subsequently, the cell storage method will be described. To begin with, an exemplary structure of the cell culture chamber will be described with reference to Figs. 1 and 2. Fig. 1 is a plane view showing the structure of the cell culture chamber according to this embodiment, and Fig. 2 is a cross-sectional view along the line II-II in Fig. 1. As shown in Fig. 1, a cell culture chamber 10 includes microchambers 11, side walls 12, and openings 13. The plurality of side walls 12 is formed in a net shape on the culture surface of the cell culture chamber 10, and spaces surrounded by the side walls 12 serve as the microchambers 11. Additionally, each of the openings 13 is formed at a central portion of each side of the side walls 12 which are formed on four sides of each of the microchambers 11.

Fig. 1 shows a width "a" of the bottom of each of the microchambers 11, a width "b" and a height "c" of each of the side walls 12 for partitioning the microchambers 11, and a width "d" of each of the openings 13 for allowing communication between the microchambers 11 adjacent to each other. The term "bottom area" of the present invention refers to a projected area which is formed when parallel light is irradiated to the bottom of the chamber from above in the direction perpendicular to the horizontal plane of the microchmaber opening (the same plane as the top surfaces of the side walls 12). For example, if the bottom of the microchamber is U-shaped, the bottom area has a shape formed by projecting parallel light incident on the bottom from above in the direction perpendicular to the opening plane. In the case of a circle or an ellipse, a major axis of a projected bottom is a distance between intersections of a long axis which runs through the center of gravity thereof and the circumference, and a minor axis of the projected bottom is a distance between intersections of a short axis which runs through the center of gravity thereof and the circumference. In the case of a polygon, the major axis and the minor axis of the projected bottom respectively correspond to a long axis and a short axis of an extrapolated circle or an extrapolated ellipse which is set so as to minimize the difference between areas of the polygon and the extrapolated circle or the extrapolated ellipse and which runs through all vertexes of the polygon. If an extrapolated circle or an extrapolated ellipse which runs through all vertexes of the polygon cannot be traced, the major axis and the minor axis respectively correspond to a long axis and a short axis of an approximate circle or an approximate ellipse which runs through the largest number of vertexes.

The bottom shape of each of the microchambers 11 is not particularly limited, and various shapes other than a square, a circle, and a polygon can be employed. In cell culture for reproducing a liver function in vivo, the bottom area is preferably 0.01 mm$^2$ to 0.1 mm$^2$. In this case, the major axis of the bottom is preferably 1 to 1.5 times the minor axis thereof. An isotropic shape is more preferably used. If a square is employed, for example, in the case of forming a mass of liver cells having an equivalent diameter of 100 $\mu$m, the length of one side thereof is preferably 100 $\mu$m to 300 $\mu$m.

An angle formed between the horizontal plane and the side walls 12 of each of the microchambers 11 should be set to an angle at which cells are prevented from running on the microchambers. Accordingly, 50% or more of an upper portion of a side surface preferably has an angle of 80° to 90°, and more preferably, 85° to 90°.

The height "c" of each of the side walls 12 may be arvitrarily set as log as the cells cultured in the microchambers 11 are prevented from running on and moving to the adjacent microchamber 11. In the case of forming a mass of liver cells having an equivalent diameter of 100 $\mu$m, the height "c" is preferably 50 $\mu$m to 150 $\mu$m, for example.

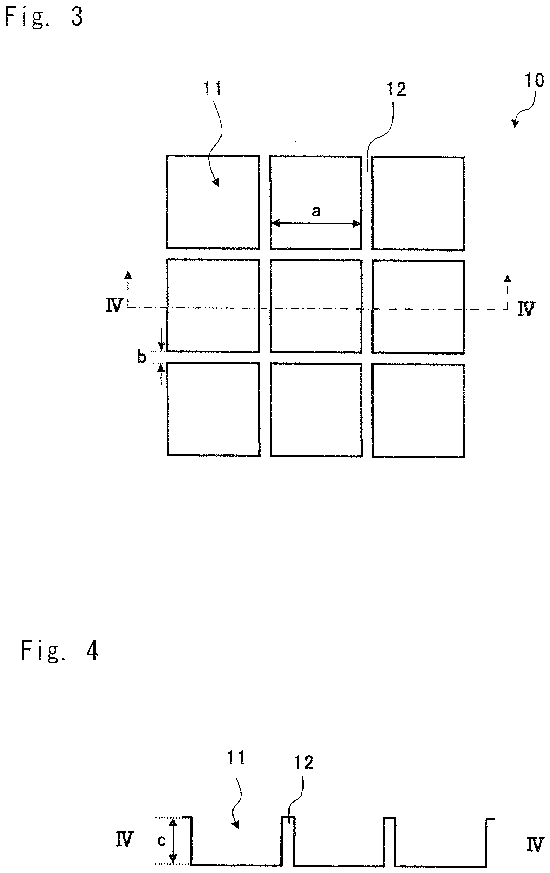

The width "d" of each of the openings 13 for allowing communication between the microchambers 11 adjacent to each other is preferably set to a width in which cells are prevented from moving from the microchamber 11, in which the cultured cell is first seeded, to the adjacent microchamber 11. When the equivalent diameter of the cultured cell is 20 $\mu$m, for example, the width is preferably 5 to 15 $\mu$m. Note that the openings 13 are not necessarily formed. As shown in Figs. 3 and 4, the four sides of each of the microchambers 11 may be entirely surrounded by the side walls 12. Here, Fig. 3 is a plane view showing another structure of the cell culture chamber according to this embodiment, and Fig. 4 is a cross-sectional view along the line IV-IV in Fig. 3.

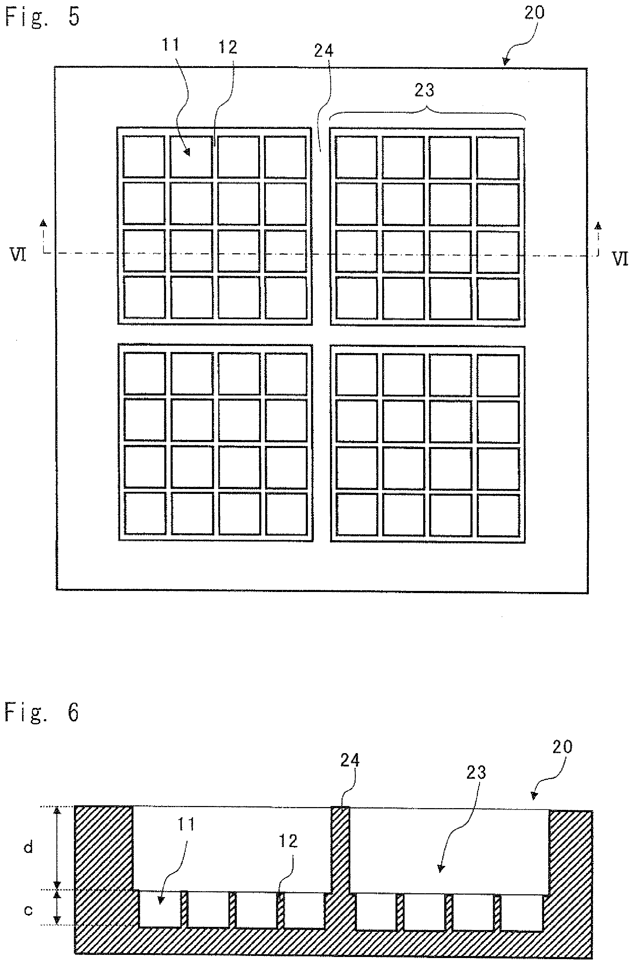

As shown in Figs. 5 and 6, the cell culture chamber according to this embodiment may have partitioned spots each made up of a given numbers of microchambers. Here, Fig. 5 is a plane view showing still another structure of the cell culture chamber according to this embodiment, and Fig. 6 is a cross-sectional view along the line VI-VI in Fig. 5. Figs. 5 and 6 show an example using the structure of the microchamber shown in Figs. 3 and 4. Fig. 5 shows side walls 24 to partition the plurality of the microchambers, and partitioned spots 23. The height "d" of each of the side walls 24 may be arbitrarily set to satisfy a capacity for storing a supernatant fluid such as culture solution or reaction solution. Since the side walls 24 are provided, different culture mediums can be used in each of the spots 23. Though Figs. 5 and 6 show an exemplary structure including the side walls 24, a structure without the side walls 24 may also be employed.

A method for forming the concave-convex pattern on the cell culture chamber is not particularly limited, but methods such as transfer molding using a mold, three-dimensional stereolithography, precision machining, wet etching, dry etching, laser processing, and electrical discharge machining may be employed. It is preferable to appropriately select these production methods in view of the intended use, required processing accuracy, costs, and the like of the cell culture chamber.

As a specific example of the transfer molding method using a mold, a method for forming the concave-convex pattern by resin molding using a metal structure as a mold may be employed. This method is preferred because it is capable of reproducing the shape of the metal structure on a resin as the concave-convex pattern with a high transcription rate, and because the raw material cost can be reduced by using a general-purpose resin material. Such a method using a mold of a metal structure is superior in terms of low cost and achieving satisfactorily high dimensional accuracy.

As methods of producing the metal structure, for example, plating treatment, precision machining, wet etching, dry etching, laser processing, and electrical discharge machining on a resist pattern produced by photolithography or a resin pattern produced by three-dimensional stereolithography may be employed. The methods may be appropriately selected in view of the intended use, required processing accuracy, costs, and the like.

As methods of forming the concave-convex pattern on a resin using the metal structure, which is obtained as described above, as a mold, injection molding, press molding, monomer casting, solvent casting, hot embossing, or roll transfer by extrusion molding may be employed, for example. It is preferable to employ injection molding in view of its productivity and transcription property.

Materials for forming a cell culture chamber are not particularly limited as long as the materials have self-supporting properties. For example, synthetic resin, silicon, or glass may be employed. A transparent synthetic resin is preferably used as a material in view of costs and cell visibility under microscopical observation. Examples of the transparent synthetic resin include acrylic resins such as polymethylmethacrylate or methyl methacrylate-styrene copolymer, styrene resin such as polystyrene, olefin resin such as cycloolefin, ester resins such as polyethylene terephthalate and polylactic acid, silicone resin such as polydimethylsiloxane, and polycarbonate resin. These resins may contain various additives such as colorant, dispersing agent, and thickening agent, unless the transparency is impaired.

In the cell culture chamber, surface treatment may be performed on the surface side of the concave-convex pattern and a modified layer and/or a coating layer may be formed for the purpose of improving the hydrophilic properties, biocompatibility, cellular affinity, and the like of the chamber surface. A method for forming the modified layer is not particularly limited unless a method with which the self-supporting properties are impaired and a method causing extreme surface roughness of 100 $\mu$m or more are employed. Methods, for example, chemical treatment, solvent treatment, chemical treatment such as introduction of a graft polymer by surface graft polymerization, physical treatment such as corona discharge, ozone treatment, or plasma treatment may be employed. In addition, though a method for forming the coating layer is not particularly limited, methods, for example, dry coating such as sputtering or vapor deposition and wet coating such as inorganic material coating or polymer coating may be employed. In order to pour a culture solution without mixing air bubbles therein, it is desirable to impart the hydrophilic properties to the surface of the concave-convex pattern. As a method for forming a uniform hydrophilic membrane, inorganic vapor deposition is preferably employed.

When the cellular affinity is taken into consideration, it is more preferable to coat cytophilic proteins such as collagen and fibronectin. In order to uniformly coat a collagen aqueous solution or the like, it is preferable to perform the coating after the above-mentioned hydrophilic membrane is formed. In hepatocyte cultures, in general, it is desirable to culture cells on an extracellular matrix surface by replicating the in vivo environment. Accordingly, it is particularly preferable to dispose an organic film made of extracellular matrix suitable for cultured cells after an inorganic hydrophilic membrane is uniformly formed as described above.

In a cell culture method using the cell culture chamber described above, an appropriate number of cells need to be seeded so that the cells are arranged exclusively within the microchambers for culturing cells, and morphologies and functions similar to those of the living body are developed within the space. A cell seeding density of $1.0 \times 10^2$ to $1.0 \times 10^6$ cells/cm$^2$ is preferably used and a cell seeding density of $1.0 \times 10^4$ to $1.0 \times 10^6$ cells/cm$^2$ is more preferably used. When each microchamber is a square which is 200 $\mu$m on a side, for example, a cell seeding density of $5.0 \times 10^4$ to $5.0 \times 10^5$ cells/cm$^2$ is preferably used. Under such conditions, a mass of liver cells having a diameter of 30 to 200 $\mu$m can be obtained.

Subsequently, the cell storage method according to this embodiment will be described. The cell storage method is a method to culture living cells by using the cell culture chamber including the plurality of culture microchambers described above and to store it. Specifically, first, a culture process is carried out in which the living cells are adhered to the surfaces of the plurality of culture microchambers of the cell culture chamber to culture the cells. Next, a storage preparation process is carried out in which a culture medium (culture solution) is poured into the cell culture chamber so as to cover the plurality of microchambers to prepare for the storage. The cell culture chamber prepared as described above is stored (storage process). Each process will be described in more detail below.

First, the culture process will be described. Adhesive cells are used as the living cells. The living cells are adhered to the surfaces of the microchambers, thereby preventing the living cells from colliding with the surfaces of the microchambers, or flowing out from the microchambers, for example, during the storage and transportation. This inhibits loss of the functions of the cultured living cells, and prevents the living cells from drying due to being exposed from the culture medium and from degradation of the cell functions due to the dry state.

Parenchymal cells are used as the living cells to be cultured. Specifically, any one of liver cells, beta cells of pancreas, cardiac muscle cells, nerve cells, skin epidermal cells, cartilage cells, bone cells, tissue stem cells, ES cells, and iPS cells (induced pluripotent stem cells) are used. Alternatively, any one of liver cells, beta cells of pancreas, cardiac muscle cells, nerve cells, skin epidermal cells, cartilage cells, and bone cells, which are differentiated from one of tissue stem cells, ES cells, and iPS cells.

The culture process includes (1) a step of adhering cells, (2) a step of removing non-adhered cells, and (3) a step of further culturing cells to allow the cells to grow, elongate, or aggregate. Removal of the non-adhered cells makes it possible to remove waste products and to prevent the culture medium from being contaminated. Additionally, the living cells are cultured by culture growth or the like until the mass of living cells reaches a desired size. For example, the living cells are allowed to grow, elongate, and aggregate until the diameter of a cellular aggregate reaches 30 to 200 $\mu$m. Each of the microchambers is designed to be large enough to contain the desired cell mass. As just described, in the culture of the living cells, the cells are cultured so that the living cells are adhered (seeded) onto the surfaces of the plurality of the microchambers, and the cell population of the three-dimensional structure formed within the space of each of the microchambers has a number of cells that are isolated from other cells.

According to the steps as described above, a cell population which corresponds to a number of cells of a single three-dimensional structure is formed within each microchamber and is isolated form other cells. Here, the cell population (cell mass) formed within each microchamber corresponds to a single three-dimensional structure. The living cells are isolated by the walls of the microchambers having a height greater than the size of the living cells and the living cells are adhered to the microchambers. Therefore, the living cells are prevented from coming into contact with adjacent three-dimensional structures. This allows the living cells to form, or maintain a uniform three-dimensional structure even under a non-static circumstance (such as a transported state).

Next, the storage preparation process will be described. The amount of culture medium to be poured is determined so as to prevent the cultured living cells from drying or contacting outside air. The culture medium for use in the storage is a medium containing nutrient components, for example, a blood serum or a component of blood, such as a growth factor. Components contained in the culture medium are determined depending on the living cells to be stored.

Further, the cell culture chamber is sealed by seal means so as to prevent the culture medium from leaking from the cell culture chamber. The cell culture chamber has a structure in which the plurality of microchambers is formed on, for example, a petri dish, a plate, or a flask having an opening portion. In the sealing process, the opening portion formed in the upper surface of the cell culture chamber is covered by the seal means, such as a film or a cap, and the living cells are stored in the state of being separated from the outside. The seal means may be made of a material that blocks a flow of liquid or gas, or a material that allows carbon dioxide or oxygen to permeate. This may be selected depending on the living cells to be stored or a culture state. Additionally, in the case of transporting the living cells, it is necessary that the culture medium is sealed so as not to be leaking from the chamber. On the other hand, during a storage in a static state, it may be allowable for closing a lid so as to prevent from drying the culture medium, or for storing with the opening portion opened. Depending on the stored state of the living cells, the cell culture chamber is not necessarily sealed.

Finally, the storage process will be described. In the storage process disclosed herein, the cell culture chamber may be adjusted to a temperature within a temperature range equal to or higher than 4°C and lower than 37°C, or a temperature range equal to or higher than 6°C and lower than 25°C. According to the present invention, a temperature range from 10°C to 20°C is used, as defined in the claims. Additionally, the storage process also includes a period for transporting the cell culture chamber which is produced and sealed in the store preparation process. It is preferable to carry out temperature adjustment also during the transportation period.

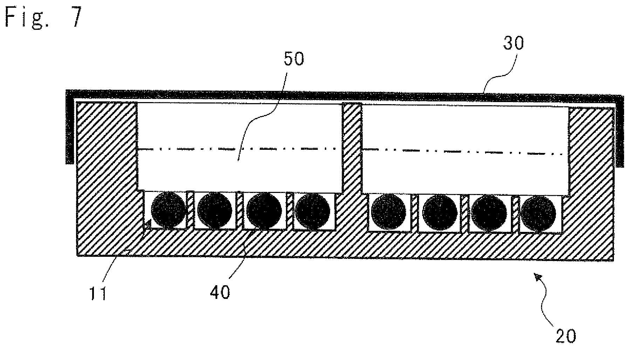

Fig. 7 shows an example of a state of the cell culture chamber after the storage preparation process. Fig. 7 shows an example where the culture process and the storage preparation process are carried out by using the cell culture chamber shown in Figs. 5 and 6 and living cells 40 and seal means 30 are added to the sectional view shown in Fig. 6. Specifically, the living cells 40 are adhered to each of the microchambers 11 within a cell culture chamber 20 and cultured. In Fig. 7, the living cells 40 are simply represented as a black circles, and a culture medium 50 is filled up to a level indicated by a two-dot chain line. The seal means 30 seals the cell culture chamber 20. The amount of culture medium shown in Fig. 7 is an example. Alternatively, an amount of culture medium corresponding to on-third of the volume of the chamber may be poured, or the culture medium may be poured until there is no airspace between the seal means 30 and the culture medium. The volume of the culture medium is arbitrarily selected depending on the kinds of living cells, culture state, or the like.

As described above, according to an aspect of the embodiment, it is possible to allow the living cells to form the three-dimensional structures adhered to a culture base made of hard plastic, without using any particular polymers, and to be stored.

It is known that when living cells form a three-dimensional structure, the functions of the cells are improved. Therefore, it is possible to store the living cells while maintaining the functions thereof, after the living cells are cultured.

Further, when the cells are transported by transportation means, such as a vehicle, a ship, or a an aircraft, there is a concern that the functions of cells are further deteriorated due to environmental factors during transportation, such as shaking, temperature, and $CO_2$ concentration (pH change in the culture medium), and that cell death is caused by cell ablation or breakup of a gel due to shaking occurring during transportation. As to these problems, it is possible to transport living cells while maintaining the functions of the living cells, by applying an aspect of the present invention.

For example, the present invention is applicable when fresh liver cells isolated from a living body are transported. Specifically, it is considered that the fresh liver cells isolated from a living body can live for only a predetermined period of time (60 hours). Even in such a case, it is to be expected that the three-dimensional structures of fresh liver cells can be maintained, and that the cells can be transported while maintaining the functions. Further, an aspect of the present invention is applicable when the fresh liver cells isolated from a living body are transported in a frozen state. That is, after melting the frozen cells, it is to be expected that the cells can be stored or transported in a non-frozen state by employing the method of the present invention. Furthermore, when the three-dimensional structures of the the living cells were transported by using polymers, such as gel, there were cases where the three-dimensional structures of the living cells was broken due to breakup of gel from the living cells or a change in shape of the gel. It is to be expected that the three-dimensional structures can be maintained, and that the living cells can be transported while maintaining the functions thereof, by applying an aspect of the present invention.

[Example]

Next, examples of the cell culture method according to the present invention are described hereinafter.

[Example 01]

Example showing a case where cells are stored using a cell culture chamber including a plurality of micro spaces and a case where cells are stored using a flat plate.

(Culture Chamber)

<Example 01>

A pattern which has the shape of the concave-convex pattern as shown in Figs. 3 and 4 and which has dimensions of a=200 $\mu$m, b=20 $\mu$m, and c=50 $\mu$m was produced by photolithography, and Ni electrolytic plating was carried out to obtain a mold having a corresponding concave-convex shape. The concave-convex pattern shape was transcribed on polystyrene by hot embossing with the mold, and a resin base material having the above-mentioned dimensions was produced. A film was produced by forming a silicon dioxide film with a thickness of 100 nm on the surface of the resin base material by vacuum deposition. The film was attached to a 24-hole plate having no culture bottom and made of polystyrene, and $\gamma$-ray sterilization was carried out to produce the culture chamber including the plurality of 24-well micro spaces for use in a storage test.

<Comparative Example 01>

A commercially available (Falcon (Registered Trademark) available from Becton, Dickinson and Company) $\gamma$-ray sterilized flat 24-well culture plate was used for the storage test.

(Cell Culture)

Human liver carcinoma cell line HepG2 cells (Japan Health Sciences Foundation, Health Science Research Resources Bank resource number JCRB1054) were grown to a predetermined number of cells in an incubator at 37°C and 5% $CO_2$ using a culture flask (manufactured by Corning Incorporated). A DMEM culture medium containing 10% fetal bovine serum (manufactured by GIBCO) was used as the culture medium. The grown cells were detached from the culture bottoms using a 0.25% trypsin solution and collected by a centrifugal separation method. The collected cells were adjusted to a cell concentration of $4 \times 10^5$ cells/ml by using a DMEM culture solution containing 10% FBS which was added to each well by 500 $\mu$l. After that, the cells were cultured in the incubator at 37°C and 5% $CO_2$ for three days.

(Storage Test)

The cells were cultured in the incubator at 37°C and 5% $CO_2$ using the culture chambers shown in Example 01 and Comparative Example 01. After that, the culture medium was vacuumed and the DMEM culture medium containing 10% FBS was newly added to each well by 500 $\mu$l. After the culture chamber was sealed with a plastic film, the culture chamber was put into an incubator at a temperature of 37°C and stored for 24 hours.

(Analysis: Measurement of Albumin Secretion)

After the storage for 24 hours, the culture medium was vacuumed and the DMEM culture medium containing 10% FBS was added to each well. Then, the cells were cultured in the incubator at 37°C and 5% $CO_2$ for two days. Cultured supernatant was collected, and a human albumin secretion for two days was measured by human albumin analysis ELISA kits (manufactured by Bethyl Laboratories, Inc.).

(Result)

Table 1 shows the result of measurement of albumin secretion. The secretion shown in Example 01 is three times the secretion of Comparative Example 01.

[Table 1]

|  | Albumin Secretion [pg/ml/2 days] |
| --- | --- |
| Example 01 | 360 |
| Comparative Example 01 | 120 |

<Examples 02 to 05>

Examples given in terms of storage temperature

(Culture Chamber)

A pattern which has the shape of the concave-convex pattern as shown in Figs. 3 and 4 and which has dimensions of a=200 $\mu$m, b=20 $\mu$m, and c=50 $\mu$m was produced by photolithography, and Ni electrolytic plating was carried out to obtain a mold having a corresponding concave-convex shape. The concave-convex pattern shape was transcribed on polystyrene by hot embossing with the mold, and a resin base material having the above-mentioned dimensions was produced. A film was produced by forming a silicon dioxide film with a thickness of 100 nm on the surface of the resin base material by vacuum deposition. The film was attached to a 24-hole plate having no culture bottom and made of polystyrene, and $\gamma$-ray sterilization was carried out to produce the culture chamber (culture plate) including the plurality of 24-well micro spaces for use in a storage test.

(Cell Culture)

Human liver carcinoma cell line HepG2 cells(Japan Health Sciences Foundation, Health Science Research Resources Bank resource number JCRB1054) were grown to a predetermined number of cells in an incubator at 37°C and 5% $CO_2$ using a culture flask (manufactured by Corning Incorporated). A DMEM culture medium containing 10% fetal bovine serum (manufactured by GIBCO) was used as the culture medium. The grown cells were detached from the culture bottoms using a 0.25% trypsin solution and collected by a centrifugal separation method. The collected cells were adjusted to a cell concentration of $4\times10^5$ cells/ml by using a DMEM culture solution containing 10% FBS which was added to each well of the culture chamber by 500 $\mu$l. After that, the cells were cultured in the incubator at 37°C and 5% $CO_2$ for three days.

(Storage Test)

After culturing the cells for three days, the culture medium was vacuumed and the DMEM culture medium containing 10% FBS was newly added to each well 500$\mu$l. The culture chamber was sealed with a plastic film and was then stored by the storage method according to the following Examples. Temperature inside the container was measured by a temperature sensor, and it was checked whether the temperatures were maintained at a target temperature.

<Example 02>

A storage container (manufactured by Hitachi Transport System, Ltd.) containing a lagging material for keeping the container at 18°C was used. The culture chamber was stored in the storage container for 24 hours. The temperature inside the storage container during this period was 18°C$\pm$0.5°C.

<Example 03>

A storage container (manufactured by Hitachi Transport System, Ltd.) containing a lagging material for keeping the container at 6°C was used. The culture chamber was stored in the storage container for 24 hours. The temperature inside the storage container during this period was 6°C$\pm$0.5°C.

<Example 04>

The culture chamber was put on a container made of Styrofoam and containing ice and stored for 24 hours. The temperature of the bottom of the culture chamber during this period was 0°C$\pm$0.5°C.

<Example 05>

The culture chamber was put into the incubator at 37°C and stored for 24 hours.

(Analysis)

After the storage for 24 hours, the culture medium was vacuumed and the DMEM culture medium containing 10% FBS was added to each well. Then, the cells were cultured in the incubator at 37°C and 5% $CO_2$ for 24 hours. After that, observation was carried out using an inverted microscope to count the number of cell masses having a size of 30 to 200 $\mu$m in a given field. The cell mass formation rate was calculated by the following equation. The cell mass formation rate before the storage was also calculated in the same manner.

$$Cell\ Mass\ Formation\ Rate =$$

$$\frac{(The\ number\ of\ micro\ spaces\ in\ one\ field\ in\ which\ a\ cell\ mass\ is\ formed)}{(The\ number\ of\ micro\ spaces\ in\ one\ field)} \times 100\,[\%]$$

$$(Equation\ 1)$$

Where, in Equation 1, the term "The number of micro spaces in one field" refers to the number of micro spaces (microchambers) existing in one field (a given range of field). The term "The number of micro spaces in one field in which a cell mass is formed" refers to the number of micro spaces, in which a cell mass having a size of 30 to 200 $\mu$m is formed, among the micro spaces in one field.

(Result)

Table 2 shows the cell mass formation rate. The cell mass formation rate before the storage was 80 to 100%. In Example 02 (18°C), the morphology before the storage was almost completely maintained. In Example 03 (6°C), the cell mass formation rate decreases, but 60 % to 80 % of the morphology was maintained. In Example 04 (0°C), the cell mass formation rate was as low as 30%, and the morphology was hardly maintained. In Example 05 (37°C), the cell mass formation rate was 25%, which was lowest, and the morphology was hardly maintained.

From this result, it turns out that the storage temperature is preferably from 10°C to 20°C, more preferable 18°C.

[Table 2]

|  | Example 02 | Example 03 | Example 04 | Example 05 |
|---|---|---|---|---|
| Cell mass formation rate (%) | 98 | 60 | 31 | 25 |

<Examples 06 and 07>

Examples given in terms of the cell density

(Culture Chamber)

In Examples 06 and 07, the same culture chamber of Examples 02 to 05 was used.

(Cell Culture)

Human liver carcinoma cell line HepG2 cells (Japan Health Sciences Foundation, Health Science Research Resources Bank resource number JCRB1054) were grown grown to a predetermined number of cells in an incubator at 37°C and 5% $CO_2$ using a culture flask (manufactured by Corning Incorporated). A DMEM culture medium containing 10% fetal bovine serum (manufactured by GIBCO) was used as the culture medium. The grown cells were detached from the culture bottoms using a 0.25% trypsin solution and collected by a centrifugal separation method. The collected cells were adjusted to cell concentration described in Examples by using a DMEM culture solution containing 10% FBS which was added to each well of the culture chamber by 500 $\mu$l. After that, the cells were cultured in the incubator at 37°C and 5% $CO_2$ for three days.

<Example 06>

When the cells were seeded, the cell concentration was adjusted to $4 \times 10^5$ cells/ml, and the cells were stored under the storage conditions described below.

<Example 07>

When the cells were seeded, the cell concentration was adjusted to $40 \times 10^5$ cells/ml, and the cells were stored under the storage conditions described below.

(Storage Conditions)

After culture for three days, the culture medium was vacuumed and the DMEM culture medium containing 10% FBS was added to each well by 500$\mu$l. The culture chamber was sealed with a plastic film. After that, the culture chamber was put into a storage container (manufactured by Hitachi Transport System, Ltd.) containing a lagging material for keeping the container at 18°C, and was stored for 24 hours.

(Analysis)

(Albumin Analysis)

After the storage for 24 hours, the culture medium was vacuumed and the DMEM culture medium containing 10% FBS was added to each well. Then, the cells were cultured in the incubator at 37°C and 5% $CO_2$ for two days. Cultured surpernatant was collected, and a human albumin secretion for two days (ng/ml/2 days) was measured by human albumin analysis ELISA kits (manufactured by Bethyl Laboratories, Inc.). Next, the cells were detached by using a 0.25% trypsin solution, the values of the living cells were calculated using trypan blue. Table 3 below shows values per $10^5$ cells as albumin secretion (pg/$10^5$/2 days).

(Morphology Observation)

After the storage for 24 hours, the culture medium was vacuumed and the DMEM culture medium containing 10% FBS was added to each well. Then, the cells were cultured in the incubator at 37°C and 5% $CO_2$ for 24 hours in the incubator at 37°C and 5% $CO_2$. After that, the morphology was observed by using an inverted microscope.

(Result)

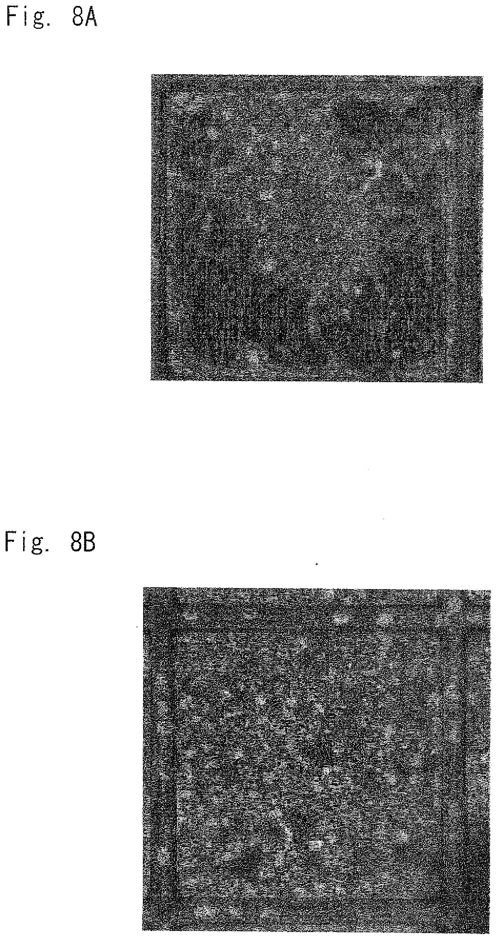

Figs. 8A and 8B are photographs taken when the cells were cultured in the incubator at 37°C and 5% $CO_2$ for 24 hours after the plate was extracted from the storage container.

In Example 06 (Fig. 8A), spherical cell masses were formed. On the other hand, in Example 07 (Fig. 8B), the cells were densely packed in each micro chamber to form cell masses, and dead cells aggregate outside the partitions. Table 3 shows albumin secretions. In terms of the albumin secretion, Example 06 shows a value as high as about 2.8 times that of Example 07.

[Table 3]

|  | Albumin Secretion [pg/$10^5$/2 days] |
|---|---|
| Example 06 | 177 |
| Example 07 | 62.5 |

[Reference Signs List]

| 10, | 20 | CELL CULTURE CHAMBER |
|---|---|---|
| 11 | | MICROCHAMBER |
| 12 | | SIDE WALL |
| 13 | | OPENING |
| 23 | | SPOT |
| 24 | | SIDE WALL OF SPOT |
| 30 | | SEAL MEANS |
| 40 | | LIVING CELL |
| 50 | | CULTURE MEDIUM |

**Claims**

**1.** A cell storage method to store living cells by using a cell culture chamber including a plurality of micro spaces wherein the plurality of micro spaces have a bottom area of 0.01 to 0.1mm$^2$ and a depth of 25 to 150$\mu$m, the cell storage method comprising the steps of:

culturing the living cells by being adhered to surfaces of the plurality of micro spaces, wherein the culturing is carried out so that a cell population of a three-dimensional structure which is adhered to the surfaces and formed within each of the micro spaces has a number of cells that are separated from other cells;
removing non-adherent cells after the culturing; and then
pouring a culture medium into the cell culture chamber so as to cover the plurality of micro spaces; and
sealing the cell culture chamber so as to prevent the culture medium from leaking from the cell culture chamber, and storing the living cells
wherein the cell culture chamber is maintained at a temperature equal to or higher than 10°C and lower than 20°C to store the living cells, and
wherein the living cells are one of liver cells, beta cells of pancreas, cardiac muscle cells, nerve cells, skin epidermal cells, cartilage cells, bone cells, tissue stem cells, ES cells, and iPS cells.

2. The cell storage method according to Claim 1 wherein the living cells are liver cells.

3. The cell storage method according to Claim 1, wherein
the living cells are one of liver cells, beta cells of pancreas, cardiac muscle cells, nerve cells, skin epidermal cells, cartilage cells, and bone cells, which are differentiated from one of tissue stem cells, ES cells, and iPS cells.

4. The cell storage method according to any one of Claims 1 to 3, wherein
the culturing of the living cells includes further culturing the living cells to allow the living cells to grow, elongate, or aggregate after the living cells are adhered and cultured.

5. The cell storage method according to any one of Claims 1 to 4, wherein
the living cells are seeded in the plurality of micro spaces at a cell seeding density of 1x10$^2$ to 1x10$^6$ cells/cm$^2$.

6. The cell storage method according to any one of Claims 1 to 5, wherein
a cell mass having the living cells accumulated therein is formed in each of the plurality of micro spaces.

7. The cell storage method according to Claim 6, wherein
the cell mass has a diameter of 30 to 200 $\mu$m.

8. The cell storage method according to any one of Claims 1 to 7, wherein
the culture medium includes at least one of a blood serum, a growth factor, and a component of blood.

9. The cell storage method according to any one of Claims 1 to 8, wherein
the cell culture chamber is sealed by using a membrane which allows oxygen or carbon dioxide to permeate.

10. Use of the method of Claim 1 for transporting living cells in vehicle, ship or aircraft.


**Patentansprüche**

1. Zelllagerungsverfahren zum Lagern von lebenden Zellen unter Verwendung einer Zellkulturkammer, die eine Vielzahl von Mikroräumen umfassen, wobei die Vielzahl von Mikroräumen einen Bodenbereich von 0,01 bis 0,1 mm$^2$ und eine Tiefe von 25 bis 150 $\mu$m aufweisen, wobei das Zelllagerungsverfahren die Schritte umfasst:

Züchten der lebenden Zellen dadurch, dass sie an die Oberflächen der Vielzahl von Mikroräumen adhäriert sind, wobei das Züchten so durchgeführt wird, dass eine Zellpopulation einer dreidimensionalen Struktur, die an die Oberflächen adhäriert ist und in jedem der Mikroräume gebildet wird, eine Anzahl an Zellen aufweist, die von anderen Zellen getrennt sind;
Entfernen von nicht-adhärierten Zellen nach dem Züchten; und anschließend Gießen eines Kulturmediums in die Zellkulturkammer, so dass die Vielzahl der Mikroräume bedeckt ist; und
Verschließen der Zellkulturkammer, so dass verhindert wird, dass das Kulturmedium aus der Zellkulturkammer austritt, und Lagern der lebenden Zellen,
wobei die Zellkulturkammer bei einer Temperatur aufrechterhalten wird, die gleich oder höher als 10°C und niedriger als 20° C ist, um die lebenden Zellen zu lagern, und
wobei die lebenden Zellen eine aus Leberzellen, Betazellen aus dem Pankreas, Herzmuskelzellen, Nervenzellen, Epidermiszellen der Haut, Knorpelzellen, Knochenzellen, Gewebestammzellen, ES-Zellen und iPS-Zellen sind.

2. Zelllagerungsverfahren nach Anspruch 1, wobei die lebenden Zellen Leberzellen sind.

3. Zelllagerungsverfahren nach Anspruch 1, wobei
die lebenden Zellen eine aus Leberzellen, Betazellen aus dem Pankreas, Herzmuskelzellen, Nervenzellen, Epidermiszellen der Haut, Knorpelzellen und Knochenzellen sind, die von einer aus Gewebestammzellen, ES-Zellen und iPS-Zellen differenziert sind.

4. Zelllagerungsverfahren nach einem der Ansprüche 1 bis 3, wobei
das Züchten der lebenden Zellen das weitere Züchten der lebenden Zellen umfasst, um zu ermöglichen, dass die lebenden Zellen wachsen, elongieren oder aggregieren, nachdem die lebenden Zellen adhäriert sind und gezüchtet wurden.

5. Zelllagerungsverfahren nach einem der Ansprüche 1 bis 4, wobei
die lebenden Zellen in die Vielzahl von Mikroräumen mit einer Zellsaatdichte von $1\times10^2$ bis $1\times10^6$ Zellen/cm$^2$ gesät sind.

6. Zelllagerungsverfahren nach einem der Ansprüche 1 bis 5, wobei
eine Zellmasse, die die lebenden Zellen darin akkumuliert hat, in jedem der Vielzahl der Mikroräume gebildet wird.

7. Zelllagerungsverfahren nach Anspruch 6, wobei
die Zellmasse einen Durchmesser von 30 bis 200 $\mu$m aufweist.

8. Zelllagerungsverfahren nach einem der Ansprüche 1 bis 7, wobei
das Kulturmedium mindestens eine aus Blutserum, einem Wachstumsfaktor und einer Blutkomponente umfasst.

9. Zelllagerungsverfahren nach einem der Ansprüche 1 bis 8, wobei
die Zellkulturkammer durch Verwendung einer Membran verschlossen ist, die es ermöglicht, dass Sauerstoff oder Kohlenstoffdioxid durchdringt.

10. Verwendung des Verfahrens nach Anspruch 1 zum Transport von lebenden Zellen im Fahrzeug, Schiff oder Flugzeug.

**Revendications**

1. Procédé de stockage de cellules pour stocker des cellules vivantes au moyen d'une chambre de culture de cellules incluant une pluralité de micro-espaces où la pluralité de micro-espaces ont une aire inférieure de 0,01 à 0,1 mm$^2$ et une profondeur de 25 à 150 $\mu$m, le procédé de stockage de cellules comprenant les étapes de:

culture des cellules vivantes par le fait qu'elles sont amenées à adhérer à des surfaces de la pluralité de micro-espaces, où la culture est réalisée de sorte qu'une population de cellules d'une structure tridimensionnelle qui est amenée à adhérer aux surfaces et formée à l'intérieur de chacun des micro-espaces a un nombre de cellules qui sont séparées d'autres cellules;
retrait des cellules non adhérentes après la culture; et ensuite
déversement d'un milieu de culture dans la chambre de culture de cellules de manière à couvrir la pluralité de micro-espaces; et
fermeture de la chambre de culture de cellules de manière à empêcher le milieu de culture de s'échapper de la chambre de culture de cellules, et stockage des cellules vivantes
où la chambre de culture de cellules est maintenue à une température égale ou supérieure à 10°C et inférieure à 20°C pour stocker les cellules vivantes, et
où les cellules vivantes sont l'une de cellules hépatiques, de cellules bêta du pancréas, de cellules du muscle cardiaque, de cellules nerveuses, de cellule épidermiques cutanées, de cellules de cartilage, de cellules osseuses, de cellules souches tissulaires, de cellules ES et de cellules iPS.

**2.** Procédé de stockage de cellules selon la revendication 1 où les cellules vivantes sont des cellules hépatiques.

**3.** Procédé de stockage de cellules selon la revendication 1, où les cellules vivantes sont l'une de cellules hépatiques, de cellules bêta du pancréas, de cellules du muscle cardiaque, de cellules nerveuses, de cellules épidermiques cutanées, de cellules de cartilage et de cellules osseuses qui sont différenciées à partir de l'une de cellules souches tissulaires, de cellules ES et de cellules iPS.

**4.** Procédé de stockage de cellules selon l'une quelconque des revendications 1 à 3, où
la culture des cellules vivantes inclut en outre la culture des cellules vivantes pour permettre aux cellules vivantes de croître, de s'allonger ou de s'agréger après que les cellules vivantes soient amenées à adhérer et cultivées.

**5.** Procédé de stockage de cellules selon l'une quelconque des revendications 1 à 4, où
les cellules vivantes sont semées dans la pluralité de micro-espaces à une densité d'ensemencement de cellules de $1 \times 10^2$ à $1 \times 10^6$ cellules/cm$^2$.

**6.** Procédé de stockage de cellules selon l'une quelconque des revendications 1 à 5, où
une masse de cellules ayant les cellules vivantes accumulées à l'intérieur est formée dans chacun de la pluralité de micro-espaces.

**7.** Procédé de stockage de cellules selon la revendication 6, où la masse de cellules a un diamètre de 30 à 200 $\mu$m.

**8.** Procédé de stockage de cellules selon l'une quelconque des revendications 1 à 7, où
le milieu de culture inclut au moins l'un d'un sérum sanguin, d'un facteur de croissance et d'un composant du sang.

**9.** Procédé de stockage de cellules selon l'une quelconque des revendications 1 à 8, où
la chambre de culture de cellules est fermée au moyen d'une membrane qui permet à l'oxygène ou au dioxyde de carbone de la traverser.

**10.** Utilisation du procédé selon la revendication 1 pour transporter des cellules vivantes dans un véhicule, un navire ou un aéronef.

Fig. 1

Fig. 2

Fig. 3

Fig. 4

Fig. 5

Fig. 6

Fig. 7

Fig. 8A

Fig. 8B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1840207 A
- JP 3177610 B
- JP 7079772 A
- JP 8308562 A